# EUROPÄISCHE PATENTSCHRIFT

(11) **EP 2 283 354 B1**
(45) Veröffentlichungstag und Bekanntmachung des Hinweises auf die Patenterteilung: **30.11.2016**
(21) Anmeldenummer: 09753833.4
(22) Anmeldetag: 25.05.2009
(51) Int. Cl.: G01N 33/18, G01N 31/00

(54) **VORRICHTUNG ZUR BESTIMMUNG DES GEHALTS AN MINDESTENS EINEM OXIDIERBAREN INHALTSSTOFF IN EINER WÄSSRIGEN FLÜSSIGKEITSPROBE**
DEVICE FOR DETERMINING THE AMOUNT OF AT LEAST ONE OXIDIZABLE INGREDIENT IN AN AQUEOUS LIQUID SAMPLE
DISPOSITIF POUR DÉTERMINER LA PROPORTION D'AU MOINS UN CONSTITUANT OXYDABLE DANS UN ÉCHANTILLON DE LIQUIDE AQUEUX

(30) Priorität: 29.05.2008 DE 102008025877
(43) Veröffentlichungstag der Anmeldung: 16.02.2011
(73) Patentinhaber: Endress + Hauser Conducta GmbH+Co. KG, 70839 Gerlingen (DE)
(72) Erfinder: Bettmann, Oliver, D-65428 Rüsselsheim (DE); Kathe, Ulrich, D-71229 Leonberg (DE)
(74) Vertreter: Andres, Angelika Maria
(86) Internationale Anmeldenummer: PCT/EP2009/056258
(87) Internationale Veröffentlichungsnummer: WO 2009/144178

(56) Entgegenhaltungen:
- EP-A- 0 964 248
- DE-A1- 4 417 247
- JP-A- 2000 065 696
- JP-A- 2007 054 791
- US-A- 3 296 435
- US-A- 3 410 662
- US-A- 4 582 686

## Beschreibung

Die vorliegende Erfindung betrifft eine Vorrichtung zur Bestimmung des Gehalts an mindestens einem oxidierbaren Inhaltsstoff in einer wässrigen Flüssigkeitsprobe, welche einen Hochtemperaturreaktor zum Aufschließen der Flüssigkeitsprobe und Bildung eines Gasgemisches, welches mindestens den Inhaltsstoff als gasförmiges Oxid enthält, umfasst.

Derartige Vorrichtungen werden beispielsweise zur Bestimmung des Kohlenstoffgehalts und/oder des Stickstoffgehalts in Abwasser eingesetzt. Einige der wichtigsten in Abwässern zu bestimmenden Inhaltsstoffe sind die folgenden:
TC (Total Carbon, Gesamtkohlenstoffgehalt), der gesamte in der wässrigen Flüssigkeit enthaltene Kohlenstoff;
TOC (Total Organic Carbon, gesamter organisch gebundener Kohlenstoff), der gesamte in der wässrigen Flüssigkeit in Form von organischen Verbindungen enthaltene Kohlenstoff;
TIC (Total Inorganic Carbon, gesamter anorganisch gebundener Kohlenstoff), der gesamte in der wässrigen Flüssigkeit in Form von anorganischen Verbindungen enthaltene Kohlenstoff;
TN_{b} (Total Nitrogen, gesamter gebundener Stickstoff), der gesamte in der wässrigen Flüssigkeit enthaltene gebundene Stickstoff.

Bei bekannten Verfahren wird eine Flüssigkeitsprobe von einem geringen Volumen von beispielsweise einigen 100 µl dem Hochtemperatrureaktor zugeführt. Im Falle der Bestimmung des TOC erfolgt gegebenenfalls eine Vorbehandlung zum Entfernen der anorganischen Verbindungen, z.B. durch Ansäuern, wobei Kohlendioxid (CO₂) entsteht und durch Ausblasen entfernt wird. Im Hochtemperaturreaktor werden die organischen Inhaltsstoffe thermisch zu CO₂, die Stickstoff enthaltenden Inhaltsstoffe zu Stickstoffoxid NOₓ aufgeschlossen. Die Kurzschreibweise NOₓ steht für ein Gemisch aus Stickoxiden mit Stickstoff in verschiedenen Oxidationsstufen, das jedoch als Hauptkomponente, nämlich zu ca. 98%, NO aufweist. In geringer Menge enthält das Gemisch auch NO₂, jedoch dauert die Umsetzung des NO in NO₂ unter den vorliegenden Bedingungen verhältnismäßig lang. Bei der Umsetzung im Hochtemperaturreaktor entsteht ein Gasgemisch, das neben CO₂ und NOₓ gasförmiges H₂O, sowie Verunreinigungen, beispielsweise durch sublimierte Salze oder durch Metalloxide, enthält. Das Gasgemisch wird mit Hilfe eines Trägergases, das in der Regel auch den nötigen Reaktionssauerstoff liefert, durch einen Kühler mit Wasserabscheider, einen Gasfilter und eine Analyseeinheit transportiert. Die Menge des entstandenen CO₂ bzw. NOₓ, wird beispielsweise durch Infrarotmessung oder durch Chemilumineszenzmessung bestimmt, und aus diesem Wert wird der TOC bzw. TN_{b} errechnet.

Für flüssige Proben beträgt die Reaktionstemperatur üblicherweise ca. 680°C bis 1000°C, wobei höhere Temperaturen den Probenaufschluss prinzipiell begünstigen. Die vollständige Umsetzung der in der Probe enthaltenen Kohlenstoff- bzw. Stickstoff-Verbindungen wird durch Einsatz von Katalysatoren, beispielsweise Platin, bzw. Sauerstoffüberträgern, z.B. Ceroxid oder Nickeloxid, unterstützt. Es ist auch möglich, den Aufschluss der Probe ohne Katalysator oder Sauerstoffüberträger durchzuführen. In diesem Fall wird die Reaktion in der Regel bei Temperaturen von mehr als 1000°C durchgeführt.

In den Flüssigkeitsproben und damit auch entsprechend in dem im Hochtemperaturreaktor anfallenden Gasgemisch kommen häufig Matrixkomponenten wie Salze, z.B. Chloride, Sulfate oder Phosphate, die bei den in der Reaktionszone des Hochtemperaturreaktors herrschenden Temperaturen durch Sublimation in die Gasphase übergehen, Metalloxide, die als feste Partikel innerhalb des Gasgemisches vorliegen können, sowie Säuren oder basische Komponenten vor. Diese Matrixkomponenten können die Bestimmung der oxidierbaren Inhaltsstoffe stören oder gar verfälschen. Beispielsweise können sich in kalten Bereichen der Vorrichtung resublimierte Salze und Metalloxidpartikel aus dem Gasgemisch absetzen und zur Zusetzung von engen Gasleitungen oder Filtern führen. Aus den Salzen oder in der Gasphase befindlichen Komponenten können außerdem korrosive Gase, Dämpfe oder Aerosole entstehen und durch das Trägergas in die vom Trägergas durchströmten Bereiche der Vorrichtung verteilt werden. Dadurch können empfindliche Teile der Apparatur, insbesondere die zur Analyse vorhandenen Detektoren, beschädigt werden.

Aus dem Stand der Technik sind Maßnahmen bekannt, diese schädlichen Auswirkungen der Matrixkomponenten, insbesondere von Salzen, zu reduzieren.

So zeigt die DE 44 17 247 B4 eine Analysevorrichtung mit einem Verbrennungsofen für Flüssigkeitsproben zur Bestimmung des Gehalts an oxidierbaren Inhaltsstoffen, in dem ein Einsatz angeordnet ist, der Füllkörper aus Quarzglas oder keramischem Material enthält. Der Einsatz ist in einem Bereich des Verbrennungsofens angeordnet, in dem die Betriebstemperatur zwischen 100°C und 400°C beträgt. Bei diesen Temperaturen schlagen sich die im Gasgemisch vorhandenen Salze an den Füllkörpern nieder und gelangen auf diese Weise nicht in die übrigen Bereiche der Analysevorrichtung.

Von Zeit zu Zeit muss der Einsatz jedoch ausgewechselt oder ausgebaut und gereinigt werden, da sein Aufnahmevermögen für die niedergeschlagenen Salze begrenzt ist. Dies erfordert eine aufwändige Dichtungstechnik, insbesondere da die Dichtflächen an der Position des Einsatzes im Verbrennungsofen für hohe Temperaturen ausgelegt sein müssen. Während des Austauschs des Einsatzes muss der Verbrennungsofen abgekühlt werden, was zu einer Ausfallzeit der Analysevorrichtung führt. Beim Herausnehmen des auszutauschenden oder zu regenerierenden Einsatzes kann es weiterhin dazu kommen, dass Salzpartikel aus dem Einsatz herausfallen und die Apparatur verschmutzen. Besonders schädlich ist es, wenn die Salzpartikel auf die Dichtflächen gelangen, da dann eine zuverlässige Abdichtung bei erneuter Inbetriebnahme nicht mehr gewährleistet ist.

In US 4,078,894 ist eine Vorrichtung zur Abwasseranalyse mit einem Hochtemperaturreaktor gezeigt, in dem eine Flüssigkeitsprobe durch Erhitzen in Anwesenheit eines als Oxidationsmittel wirkenden Trägergases zu einem Gasgemisch umgesetzt wird. Der Gasauslass des Reaktors mündet in eine Kammer, die in einem unteren Bereich mit Wasser gefüllt ist, und in der das Gasgemisch so weit abgekühlt wird, dass darin enthaltene Salze abgeschieden und im Wasser gelöst werden. Durch Abpumpen des Wassers aus der Kammer werden die Salze aus dem System entfernt. In Zusammenwirkung mit dem mit hoher Strömungsgeschwindigkeit an dem Kondensat vorbeiströmenden Gasstrom können bei einer derartigen Vorrichtung jedoch schädliche Aerosole entstehen.

In JP 2007-054791 A ist eine Vorrichtung zur Aufbereitung von Abwasser mittels Verbrennung gezeigt, an die ein Analysegerät zur Bestimmung verschiedener Inhaltsstoffe im Prozessabgas anschließbar ist. Um die Messung nicht zu verfälschen, soll vor der Einleitung des Prozessabgases in das Analysegerät salzhaltiger Nebel bzw. Aerosole aus dem Prozessabgas entfernt werden. Dies erfolgt mittels einer Gasreinigungseinheit (Scrubber) mit einem in Reihe geschalteten Feinfilter, die gemeinsam in einem Gehäuse angeordnet sind. Das Gehäuse wird auf eine Temperatur oberhalb der Kondensationstemperatur des Wassergehalts im Prozessabgas und unterhalb von 150°C erwärmt, um die Auskondensation von Wasser aus dem Prozessabgas zu verhindern. Die Entfernung von Aerosolen aus einer Vorrichtung ist jedoch mit verhältnismäßig hohem apparativem Aufwand verbunden.

Aufgabe der Erfindung ist nun sicherzustellen, dass im Gasstrom vor dem Erreichen der Filtereinheit keine Kondensation erfolgt und den Gasauslass, die Filtereinheit, und zwischen der Filtereinheit und dem Gasauslass vorhandene Verbindungselemente, wie z. B. Gasleitungen, thermisch zu isolieren und/oder mit Heizelementen auszustatten, so dass ihre Temperatur im Betrieb auf mehr als 100°C einstellbar ist. Unter dem Einstellen der Temperatur ist hier neben dem aktiven Einregeln der Temperatur auch ein Aufrechterhalten einer über 100°C liegenden Temperatur allein mittels thermischer Isolierung oder durch ungeregeltes Heizen zu verstehen.

Es ist auch vorgesehen eine Vorrichtung zur Bestimmung des Gehalts an mindestens einem oxidierbaren Inhaltsstoff in einer wässrigen Flüssigkeitsprobe anzugeben, die die Nachteile der aus dem Stand der Technik bekannten Vorrichtungen überwindet. Insbesondere ist es vorgesehen, eine Vorrichtung mit einer einfach zu wartenden, insbesondere einfach austauschbaren, Filtervorrichtung anzugeben, die eine schnelle und umfassende Entfernung von Salzen aus dem Gasgemisch unter Vermeidung von Aerosolbildung sowie die Entfernung anderer im Gasgemisch möglicherweise vorhandener störender Komponenten, wie z.B. Metalloxiden, erlaubt.

Die Aufgabe wird gelöst durch eine Vorrichtung zur Bestimmung des Gehalts an mindestens einem oxidierbaren Inhaltsstoff in einer wässrigen Flüssigkeitsprobe, umfassend einen Hochtemperaturreaktor zum Aufschließen der Flüssigkeitsprobe und Bildung eines Gasgemisches, welches mindestens den Inhaltsstoff als gasförmiges Oxid enthält, wobei der Hochtemperaturreaktor einen Flüssigkeitseinlass zur Zuführung der Flüssigkeitsprobe und einen Gaseinlass zur Zuführung eines Trägergases aufweist, und über einen Gasauslass mit einer Analysekammer verbunden ist, wobei der Analysekammer eine Kondensationseinheit zur Auskondensation von Wasser aus dem Gasgemisch vorgeschaltet ist,
wobei sich im Betrieb der Vorrichtung ein Gasstrom des Trägergases mit dem Gasgemisch vom Hochtemperaturreaktor über den Gasauslass und die Kondensationseinheit bis in die Analysekammer ausbildet,
wobei zwischen dem Gasauslass und der Kondensationseinheit eine Filtereinheit zur Entfernung von Salzen und/oder Metalloxiden aus dem Gasgemisch zwischengeschaltet ist, und der Gasauslass, die Filtereinheit und zwischen Gasauslass und Filtereinheit angeordnete Verbindungselemente derart thermisch isoliert und/oder mit Heizelementen ausgestattet sind, dass ihre Temperatur im Betrieb der Vorrichtung auf mehr als 100°C eingestellt ist.

Bei Verlagerung der Filtereinheit in der Vorrichtung nach DE 44 17 247 B4 in einen im Betrieb der Vorrichtung kühleren Bereich in Gasstromrichtung hinter den Hochtemperaturreaktor wird beobachtet, dass vermehrt Aerosole im System auftreten. Dies scheint damit zusammenzuhängen, dass Teile der Vorrichtung zwischen dem Hochtemperaturreaktor und der Filtereinheit auf Temperaturen unterhalb von 100°C abkühlen, so dass an ihnen im Gasstrom vorliegende Wasser kondensiert. Im kondensierten Wasser lösen sich im Gasstrom enthaltene Salze, teilweise unter Säurebildung. Der Gasstrom bewirkt eine feine Verteilung der so gebildeten Salz- oder Säurelösung als Aerosol. In Experimenten konnte gezeigt werden, dass nur dann Aerosole in nachweisbarer Menge auftreten, wenn es zu einer Kondensation des ungefilterten Gasgemischs kommt.

Bei der hier beschriebenen Vorrichtung ist deshalb zwischen dem Gasauslass und der Kondensationseinheit eine Filtereinheit zur Entfernung der Salze aus dem Gasgemisch angeordnet. Gleichzeitig entfernt die Filtereinheit auch im Gasgemisch gegebenenfalls vorhandene beispielsweise als feste Partikel vorliegende Metalloxide oder andere Partikel aus dem Gasstrom. Durch die Anordnung der Filtereinheit zwischen dem Gasauslass und der Kondensationseinheit wird einerseits eine leichtere Auswechselbarkeit und/oder Wartung der Filtereinheit gewährleistet, da die Temperatur im Betrieb der Vorrichtung in diesem Bereich bereits deutlich geringer ist als im Hochtemperaturreaktor selbst. Andererseits werden auf diese Weise bereits vor der Auskondensation des im Gasgemisch vorhandenen Wassers Salze und/oder Metalloxide weitgehend aus dem Gasgemisch entfernt.

Die Reaktionszone erreicht im Betrieb eine Temperatur von 600°C bis zu mehr als 1000°C. Zur Unterstützung der vollständigen Umsetzung der oxidierbaren Inhaltsstoffe, beispielsweise organische kohlenstoffhaltige oder stickstoffhaltige Verbindungen, wird in der Reaktionszone des Hochtemperaturreaktors optional ein Katalysator bzw. ein Sauerstoffüberträger vorgesehen, wie beispielsweise ein Platin-Katalysator oder Ceroxid oder Nickeloxid. Die Temperatur der Reaktionszone wird dann auf einen Wert zwischen 680°C und 1000°C eingestellt. Wenn kein Katalysator oder Sauerstoffüberträger verwendet wird, wird die Reaktionszone im Betrieb in der Regel auf Temperaturen von mehr als 1000°C gebracht.

Als Trägergas wird vorzugsweise Sauerstoff oder ein sauerstoffhaltiges Gas oder Gasgemisch verwendet, das gleichzeitig als Oxidationsmittel für die zu bestimmenden Inhaltsstoffe dient. Die Vorrichtung ist so ausgestaltet, dass sich im Betrieb ein Gasstrom des im Überschuss vorhandenen Trägergases mit dem Gasgemisch vom Hochtemperaturreaktor über den Gasaulass und die Kondensationseinheit in die Analysekammer ausbildet.

Der Flüssigkeitseinlass und der Gaseinlass sind jeweils als Zuführungen an der Eingangsseite des Hochtemperaturreaktors ausgestaltet. Flüssigkeits- und Gaseinlass können auch durch eine einzelne Zuführung realisiert sein. In diesem Fall wird das Trägergas gemeinsam mit der Flüssigkeitsprobe in den Hochtemperaturreaktor eindosiert.

In einer vorteilhaften Ausgestaltung umfasst die Filtereinheit einen ersten Bereich, der dazu ausgebildet ist, Salze und/oder Metalloxide durch Resublimation und/oder Sedimentation aus dem Gasgemisch zu entfernen und einen, dem ersten Bereich nachgeschalteten, d.h. in Richtung des Gasstroms hinter dem ersten Bereich angeordneten, zweiten Bereich, der dazu ausgebildet ist, Salze und/oder Metalloxide durch Zurückhalten in einem Filtermittel aus dem Gasgemisch zu entfernen.

Unter Sedimentation wird das Absetzen von Teilchen aus dem Gasgemisch unter dem Einfluss von Kräften, insbesondere der Schwerkraft, verstanden. In einem nahe dem Gasauslass gelegenen Bereich des Hochtemperaturreaktors, im Gasauslass selbst, in gegebenenfalls vorhandenen Verbindungselementen zwischen Gasauslass und Filtereinheit sowie innerhalb der Filtereinheit selbst herrschen deutlich niedrigere Temperaturen als in der Reaktionszone des Hochtemperaturreaktors. Dort können im Gasstrom enthaltene Salze resublimieren und sich als feste Salzpartikel absetzen. Unter dem Einfluss der Schwerkraft, gegebenenfalls unterstützt durch die Verringerung der Strömungsgeschwindigkeit beim Eintritt des Gasstroms in die Filtereinheit, können sich diese Salzpartikel gegebenenfalls zusammen mit anderen im Gasstrom vorhandenen festen Partikeln, z.B. Metalloxidpartikeln, in der Filtereinheit absetzen. Der erste Bereich der Filtereinheit ist derart ausgestaltet, dass das resublimierte und sedimentierte Salz sowie sedimentierte Metalloxidpartikel mindestens zum größten Teil dort zurückgehalten werden.

Um zu verhindern, dass der Gasstrom zur Analysekammer im ersten Bereich der Filtereinheit abgesetzte Partikel aufwirbelt und weiter in Richtung der Analysekammer transportiert, ist ein zweiter Bereich der Filtereinheit dazu ausgestaltet, mit einem Filtermittel Partikel zurückzuhalten.

In einer Weiterbildung ist die Filtereinheit mittels mindestens eines Heizelements auf eine Betriebstemperatur oberhalb der Kondensationstemperatur von Wasser, insbesondere auf eine Temperatur zwischen 100°C und 150°C, bevorzugt zwischen 110°C und 130°C, beheizbar. Auf diese Weise wird die Kondensation von Wasser innerhalb der Filtereinheit vermieden.

In einer Weiterbildung sind der Gasauslass und die Filtereinheit über eine thermisch isolierte und/oder beheizte Gasleitung als Verbindungselement miteinander verbunden.

In einer vorteilhaften Weiterbildung sind der Gasauslass und/oder die Gasleitung, über die der Gasauslass mit der Filtereinheit verbunden ist, weitlumig, insbesondere mit einem Durchmesser von mehr als 3 mm, ausgebildet und derartig geformt, dass im Hochtemperaturreaktor im Bereich des Gasauslasses niedergeschlagene Salze oder andere Partikel mindestens teilweise aufgrund der Schwerkraftwirkung und/oder mit dem Gasstrom in die Filtereinheit, insbesondere in den ersten Bereich der Filtereinheit, gelangen. Auf diese Weise wird die Schwerkraft vorteilhaft ausgenutzt, um niedergeschlagene feste Partikel in die Filtereinheit zu transportieren. Gleichzeitig wird so ein Zusetzen des Gasauslasses bzw. eine Blockierung des Gasstroms durch sich ansammelnde Partikel vermieden.

In einer Weiterbildung umfasst die Filtereinheit im ersten Bereich eine gegen die Umgebung hermetisch abgedichtete Filterkammer zur Aufnahme von resublimiertem und/oder sedimentiertem Salz und/oder sedimentiertem Metalloxid.

In einer Ausgestaltung wird die Filterkammer im Wesentlichen aus einem mit dem Gasauslass oder der Gasleitung vom Gasauslass zur Filtereinheit, insbesondere über eine Schnellkupplung, lösbar verbundenen Kammerdeckel und eine lösbar mit dem Kammerdeckel verbundene Wanne gebildet. Dies hat den Vorteil, dass die Filtereinheit zur Wartung oder zum Austausch schnell und einfach aus der Vorrichtung gelöst und entfernt werden kann. Falls die Wartung nur die Leerung der Filterkammer erfordert, kann der Kammerdeckel mit dem Gasauslass verbunden bleiben und lediglich die Wanne entfernt und geleert werden.

In einer Ausgestaltung weist die Filtereinheit im zweiten Bereich einen Feinfilter auf, welcher die Filtereinheit, insbesondere das Innere der Filterkammer gegenüber der Kondensationseinheit gasdurchlässig abschließt. Der Feinfilter ist vorzugsweise an einer mit dem Kammerdeckel verbundenen Halterung befestigt. Dies hat den Vorteil, dass auch zum Austausch des Feinfilters lediglich die Filterkammer oder sogar nur die Wanne gelöst werden muss.

In einer vorteilhaften Weiterbildung umfasst der Feinfilter ein Filtermittel mit einer Struktur, welche derart ausgestaltet ist, dass die Abscheidung der Salze oder anderer im Gasstrom enthaltener Substanzen nach dem Prinzip der Tiefenfiltration im Inneren des Filtermittels erfolgt. Bei der Tiefenfiltration besteht das Filtermittel aus einzelnen Teilchen oder aus einer porösen Struktur, wobei die zurückgehaltenen Stoffe hauptsächlich innerhalb des Filtermittels, d.h. in der Porenstruktur oder an den Teilchenoberflächen adsorbiert werden. Dies hat gegenüber dem Prinzip der Kuchen- oder Oberflächenfiltration, bei der die zurückgehaltene Substanz sich auf der Oberfläche des Filtermittels als zusammenhängender Filterkuchen absetzt, den Vorteil, dass eine Zusetzung des Feinfilters langsamer erfolgt und somit die Zeitintervalle zwischen den Wartungen verlängert werden können.

Beispielsweise umfasst der Feinfilter ein PTFE-Sinterfilterelement, ein Aktivkohlefilterelement, PP-Spinnfasern oder ein Edelstahlgewebe, wobei die mittlere Porengröße des Feinfilters 0,4 bis 100 µm, insbesondere 5 bis 25 µm beträgt.

In einer vorteilhaften Ausgestaltung ist in Strömungsrichtung des Gasstroms vor dem Feinfilter, insbesondere innerhalb des Gaseinlasses zur Zuführung des Trägergases, ein Drucksensor angeordnet. Der Drucksensor ermöglicht eine Überwachung des Drucks vor der Filtereinheit. Ein Druckanstieg weist auf eine Zusetzung des Feinfilters hin. Mit der Druckmessung kann bei Überschreitung eines vorgegebenen Druck-Schwellenwertes die Ausgabe eines Warnsignals verbunden sein, das auf die Notwendigkeit einer Filterwartung hinweist.

Die Erfindung wird nun anhand eines in der Zeichnung gezeigten Ausführungsbeispiels erläutert. Es zeigt:

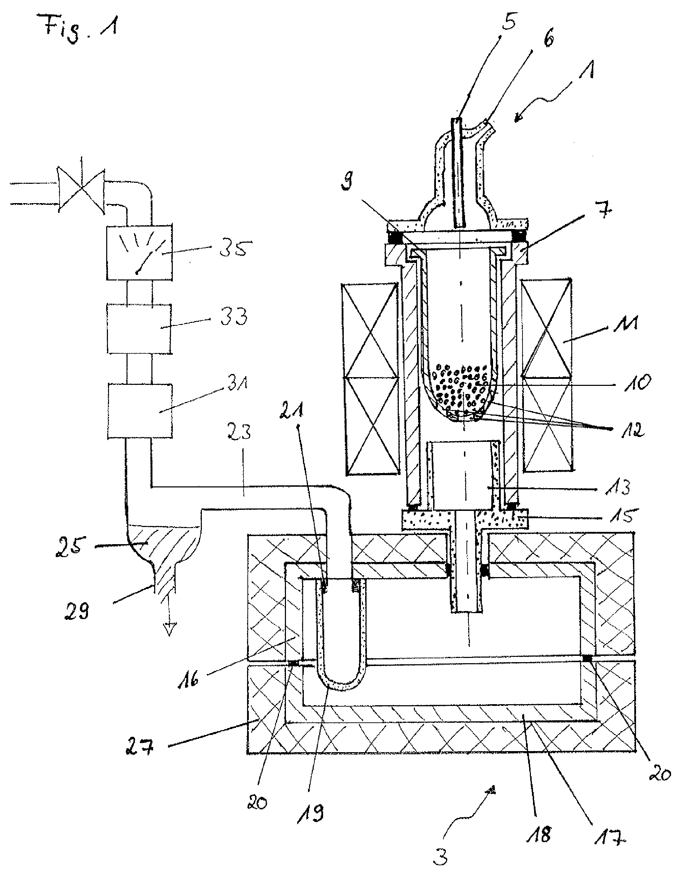

- Fig. 1: eine schematische Darstellung einer Vorrichtung zur Bestimmung des Gehalts an mindestens einem oxidierbaren Inhaltsstoff in einer wässrigen Flüssigkeitsprobe mit einer Filtereinheit.

Bei der in Fig. 1 dargestellten Vorrichtung 1 wird die zu untersuchende Flüssigkeitsprobe, beispielsweise eine Abwasser-Probe, über eine Einspritzdüse 5 einem beispielsweise als Pyrolyserohr 7 ausgeführten Hochtemperaturreaktor zugeführt. Gleichzeitig wird dem Hochtemperaturreaktor über eine weitere Zuführung 6 ein sauerstoffhaltiges Trägergas zugeführt. Das Pyrolyserohr 7 enthält einen Einsatz 9, welcher einen Katalysator 10 enthält, der die Umsetzung der Flüssigkeitsprobe mit dem sauerstoffhaltigen Trägergas unterstützt. Die Temperatur des Hochtemperaturreaktors ist mittels einer das Pyrolyserohr 7 umgebenden Heizvorrichtung 11 einstellbar. Im Bereich des Einsatzes 9 befindet sich die Reaktionszone, in der im Betrieb eine Temperatur zwischen 680°C und 1000°C herrscht. Optional kann neben dem Katalysator 10 im Einsatz 9 innerhalb der Reaktionszone weiteres Schüttgut (nicht eingezeichnet) aufgenommen sein, das durch den mit Durchtrittskanälen 12 versehenen Siebboden des Einsatzes 9 zurückgehalten wird. Im Kontakt mit dem Katalysator 10 und dem Schüttgut erhitzt sich die Flüssigkeitsprobe schnell auf die Reaktionstemperatur und wird in die Gasphase überführt, so dass keine Flüssigkeit durch den Siebboden in die übrigen Teile der Vorrichtung 1 gelangt. Unterhalb des Einsatzes 9 ist innerhalb des Pyrolyserohrs 7 eine weitere Kammer 13 angeordnet, in der im Betrieb bereits eine niedrigere Temperatur herrscht als in der Reaktionszone.

Am der Einspritzdüse 5 entgegengesetzten unteren Ende des im Betrieb der Vorrichtung 1 vertikal ausgerichteten Pyrolyserohrs 7 befindet sich ein Gasauslass 15, der im Inneren der Filterkammer 17 einer mit dem Gasauslass 15 direkt verbundenen Filtereinheit 3 mündet, so dass ein im Pyrolyserohr 7 erzeugtes Gasgemisch über die Durchtrittskanäle 12, die Kammer 13 und den Gasauslass 15 mit dem Trägergas ins Innere der Filterkammer 17 strömen kann. Die Filterkammer 17 wird im Wesentlichen durch einen Kammerdeckel 16 und eine Wanne 18 gebildet. Der Kammerdeckel 16 ist über eine Schnellkupplung (nicht gezeigt) lösbar mit dem Gasauslass 15 des Hochtemperaturreaktors verbunden. Die Wanne 18 ist lösbar, aber mittels der Dichtungen 20 hermetisch gegenüber der Umgebung abgedichtet, mit dem Kammerdeckel 16 verbunden.

Die Filterkammer 17 ist mit einer Kondensationseinheit 25 über eine Gasleitung 23 verbunden. Ein zylindrischer Feinfilter 19 schließt das Innere der Filterkammer 17 gegenüber der Gasleitung 23 gasdurchlässig ab. Der Feinfilter 19 ist über eine Halterung 21 mit dem Kammerdeckel 16 lösbar verbunden. Die Filterkammer 17 und der Gasauslass 15 des Hochtemperaturreaktors sind mit einer Isolationshülle 27 aus isolierendem Material wie beispielsweise temperaturbeständigem Schaumstoff umgeben. Optional können der Gasauslass 15 und die Filterkammer 17 mit Heizelementen (nicht dargestellt) versehen sein. Diese können beispielsweise außerhalb der Filterkammer 17 zwischen der Außenwand der Filterkammer 17 und der Isolationshülle 27 angeordnet sein. Zum Beispiel kann eine von außen auf die Filterkammer 17 aufgeklebte Heizfolie als Heizelement verwendet werden.

Ein aus dem Gasauslass 15 ins Innere der Filterkammer 17 strömender Gasstrom, der sich im Wesentlichen aus dem im Hochtemperaturreaktor entstehenden Gasgemisch und dem Trägergas zusammensetzt, kann über die Filterkammer 17 nach Passieren des Feinfilters 19 durch die Gasleitung 23 in die Kondensationseinheit 25 strömen. Die Kondensationseinheit 25 dient zur Abscheidung von Wasser aus dem Gasstrom und ist deshalb gegebenenfalls mit einem Kühler versehen, um die Kondensation aus dem Gasstrom zu beschleunigen. Das Kondensat wird über eine Leitung 29 aus der Vorrichtung 1 entfernt.

In Strömungsrichtung des Gasstroms hinter der Kondensationseinheit 25 sind eine optionale Trocknungseinheit 31, ein weiterer Filter 33 und eine Analysekammer 35 angeordnet. In der Analysekammer 35 wird der Gehalt an gasförmigem CO₂ und/oder NOₓ bestimmt. In der Regel wird zur Bestimmung des CO₂-Gehalts eine Infrarot-Messeinrichtung, z.B. ein Infrarot-Detektor verwendet. Zur Bestimmung des NOₓ-Gehalts wird in der Regel ein Chemilumineszenz-Detektor eingesetzt. Die Messsignale werden einer (nicht dargestellten) Datenverarbeitungseinheit zugeführt, die Ergebnissignale an eine (ebenfalls nicht dargestellte) Anzeige- und/oder Aufzeichnungseinheit liefert.

Die Vorrichtung 1 ist entlang des gesamten Strömungswegs des Gasstroms hermetisch gegenüber der Umgebung abgedichtet, so dass kein Gas aus der Vorrichtung 1 austreten kann.

Bei den in der Reaktionszone herrschenden Temperaturen gehen in der in den Hochtemperaturreaktor eingebrachten Flüssigkeitsprobe enthaltene Salze wie Chloride, Sulfate oder Phosphate durch Sublimation in die Gasphase über. In einem in der Nähe des Gasauslasses 15 liegenden Bereich des Pyrolyserohrs 7, zum Beispiel in der Kammer 13, herrscht jedoch bereits eine niedrigere Temperatur von 150 - 450°C, bei der zumindest ein Teil dieser Salze bereits wieder durch Resublimation in die feste Phase übergeht und sich in Form von Salzpartikeln an der Rohrwand oder im Gasauslass 15 festsetzt. Mit zunehmendem Abstand von der Reaktionszone des Hochtemperaturreaktors nimmt die Temperatur im Betrieb weiter ab. Entsprechend besitzen der Gasauslass 15 und die Filtereinheit 3 im Betrieb eine noch niedrigere Temperatur, so dass die Resublimation der in der Gasphase befindlichen Salze aus dem Gasstrom in diesen Bereichen verstärkt stattfindet. Ein Niederschlag von Salzpartikeln kann also im in der Nähe des Gasauslass 15 befindlichen Bereich des Pyrolyserohrs 7, in der Kammer 13, im Gasauslass 15 und in der Filtereinheit 3 erfolgen.

Der Gasauslass 15 des Hochtemperaturreaktors ist weitlumig ausgeführt, d.h. er besitzt einen inneren Durchmesser von mehr als 3 mm. Auf diese Weise fallen im Pyrolyserohr 7 oder im Gasauslass 15 selbst abgeschiedene Salzpartikel durch den Gasauslass 15 in die Filterkammer 17, ohne dass die Gefahr einer Zusetzung des Gasauslasses 15 besteht. Es ist zusätzlich von Vorteil, den Gasauslass 15 so zu gestalten, dass diese Salzpartikel mindestens teilweise aufgrund der Schwerkraftwirkung in die Filterkammer 17 gelangen. Dazu kann beispielsweise das Innere des röhrenförmigen Gasauslasses 15 so geformt sein, dass es mindestens einen Pfad innerhalb der Röhre gibt, der streng monoton in Richtung der Filterkammer 17 abfällt. Der Transport der Salzpartikel in die Filterkammer 17 wird nicht nur durch die Schwerkraft, sondern auch durch den Gasstrom begünstigt, der Salzpartikel erfasst und in die Filterkammer 17 transportiert. Vorteilhafterweise liegt der Durchmesser des Gasauslasses 15 im Bereich zwischen 6 und 12 mm, prinzipiell kann er aber auch genauso groß gewählt werden wie der Durchmesser des Pyrolyserohres 7.

Durch den Gasauslass 15 fallende und in der Filterkammer 17 selbst abgeschiedene Salzpartikel sammeln sich im Inneren der Filterkammer 17 an. Dies wird zusätzlich dadurch begünstigt, dass in Strömungsrichtung hinter dem Gasauslass 15 der Gasweg beim Eintritt in das Innere der Filterkammer 17 breiter wird und infolge dessen die Strömungsgeschwindigkeit sinkt. Die reduzierte Strömungsgeschwindigkeit erleichtert das Absetzen von im Gasstrom enthaltenen festen Partikeln. Die Filterkammer 17 bildet also sowohl eine Aufnahme für durch Sedimentation (Absetzen) aus dem Gasstrom entfernte Salzpartikel, als auch für Salzpartikel, die im Innern der Filterkammer 17 durch Resublimation gebildet werden. Auf die gleiche Weise wie die Salzpartikel werden auch andere gegebenenfalls im Gasstrom vorhandene feste Partikel, wie zum Beispiel Metalloxid-Partikel ins Innere der Filterkammer transportiert und durch Sedimentation aus dem Gasstrom entfernt.

In Richtung zur Analysekammer 35 passiert der Gasstrom den Feinfilter 19. Dieser dient dazu, im Gasstrom noch enthaltene und nicht abgesetzte oder durch den Gasstrom in der Filterkammer aufgewirbelte Salzpartikel zurückzuhalten. Der Feinfilter 19 umfasst als Filtermittel beispielsweise ein PTFE-Sinterfilterelement, ein Aktivkohlefilterelement, PP-Spinnfasern oder ein Edelstahlgewebe, wobei die mittlere Porengröße des Filtermittels 0,4 bis 100 µm, bevorzugt 5 bis 25 µm beträgt. Die Wirkungsweise des Filtermittels ist dadurch charakterisiert, dass die Partikel hauptsächlich im Innern der Porenstruktur des Filtermittels gebunden und zurückgehalten werden.

Um die Filtereinheit 3 zu warten, wird entweder die gesamte Filtereinheit an den Schnellkupplungen von dem Gasauslass 15 und der Gasleitung 23 abgetrennt, die Filterkammer 17 geleert und der Feinfilter 19 regeneriert oder ersetzt. Alternativ kann auch nur die Wanne 18 abgenommen und geleert werden. Nach Abnehmen der Wanne 18 ist auch der Feinfilter 19 frei zugänglich und kann von der Halterung 21 gelöst werden, um ihn auszutauschen oder zu regenerieren. Zur Überwachung der Filtereinheit 3 kann an einer im Prinzip beliebigen Stelle der Vorrichtung in Strömungsrichtung des Gasstroms vor dem Feinfilter ein Drucksensor vorgesehen werden. Vorteilhafterweise wird der Drucksensor in der Zuführung 6 für das Transportgas zum Hochtemperaturreaktor vorgesehen, da in diesem Bereich noch niedrige Temperaturen herrschen. Alternativ kann der Drucksensor aber auch im Bereich des Gasauslasses 15 oder in der Filtereinheit 3 vorgesehen werden. Detektiert der Drucksensor einen Druckanstieg, so ist dies ein Hinweis auf eine Zusetzung des Feinfilters 19, den der Gasstrom dann nicht mehr ungehindert passieren kann. Steigt der Druck über einen festgelegten Schwellenwert hinaus an, kann eine mit dem Drucksensor verbundene Datenverarbeitungs- und/oder Ausgabeeinheit einen Alarm ausgeben, der zu einer Wartung der Filtereinheit 3 auffordert.

Zur Vermeidung der Entstehung von Aerosolen ist es ein wesentlicher Bestandteil der Erfindung, wie weiter oben bereits ausgeführt, wenn es vor der Abtrennung der Salze nicht zu einer Kondensation von Wasser aus dem im Hochtemperaturreaktor aus der Flüssigkeitsprobe gebildeten Gasgemisch kommt. Um dies zu gewährleisten, ist der gesamte vom Gasstrom durchströmte Bereich zwischen der Reaktionszone im Einsatz 9 bis einschließlich der Filtereinheit 3 mittels einer Isolationshülle 17 thermisch gegenüber der Umgebung isoliert. Im Betrieb wird die Temperatur der Filtereinheit 3 sowie aller vom Gasstrom durchströmten Bereiche der Vorrichtung 1 zwischen dem Hochtemperaturreaktor und der Filtereinheit 3 auf eine Temperatur oberhalb von 100°C geregelt.

## Patentansprüche

1. Vorrichtung (1) zur Bestimmung des Gehalts an mindestens einem oxidierbaren Inhaltsstoff in einer wässrigen Flüssigkeitsprobe,
umfassend einen Hochtemperaturreaktor zum Aufschließen der Flüssigkeitsprobe und Bildung eines Gasgemisches, welches mindestens den Inhaltsstoff als gasförmiges Oxid enthält,
wobei der Hochtemperaturreaktor einen Flüssigkeitseinlass (5) zur Zuführung der Flüssigkeitsprobe und einen Gaseinlass (6) zur Zuführung eines Trägergases aufweist, und
über einen Gasauslass (15) mit einer Analysekammer (35) verbunden ist, wobei der Analysekammer (35) eine Kondensationseinheit (25) zur Auskondensation von Wasser aus dem Gasgemisch vorgeschaltet ist, wobei die Vorrichtung so ausgestaltet ist, dass sich im Betrieb ein Gasstrom des Trägergases mit dem Gasgemisch vom Hochtemperaturreaktor über den Gasauslass (15) und die Kondensationseinheit (25) in die Analysekammer (35) ausbildet,
wobei zwischen dem Gasauslass (15) und der Kondensationseinheit (25) eine beheizbare Filtereinheit (3) zur Entfernung von Salzen und/oder Metalloxiden aus dem Gasgemisch zwischengeschaltet ist,
**dadurch gekennzeichnet,**
**dass** der
Gasauslass (15), die Filtereinheit (3) und zwischen Gasauslass (15) und Filtereinheit (3) angeordnete Verbindungselemente derart thermisch isoliert und/oder mit Heizelementen ausgestattet sind, dass ihre Temperatur im Betrieb der Vorrichtung (1) auf mehr als 100°C eingestellt ist.

2. Vorrichtung nach Anspruch 1,
wobei die Filtereinheit (3) einen ersten Bereich umfasst, der dazu ausgebildet ist, Salze und/oder Metalloxide durch Resublimation und/oder Sedimentation aus dem Gasgemisch zu entfernen und einen zweiten, dem ersten Bereich nachgeschalteten, Bereich, der dazu ausgebildet ist, Salze und/oder Metalloxide, durch Zurückhalten in einem Filtermittel aus dem Gasgemisch zu entfernen.

3. Vorrichtung nach Anspruch 1 oder 2,
wobei die Filtereinheit (3) mittels mindestens eines Heizelements auf eine Betriebstemperatur oberhalb der Kondensationstemperatur von Wasser, insbesondere auf eine Temperatur zwischen 100°C und 150°C, beheizbar ist.

4. Vorrichtung nach einem der Ansprüche 1 bis 3,
wobei der Gasauslass (15) und die Filtereinheit (3) über eine thermisch isolierte und/oder beheizte Gasleitung als Verbindungselement miteinander verbunden sind.

5. Vorrichtung nach einem der Ansprüche 1 bis 4,
wobei der Gasauslass (15) und/oder die Gasleitung, über die der Gasauslass (15) mit der Filtereinheit (3) verbunden ist, weitlumig, insbesondere mit einem Durchmesser von mehr als 3 mm, ausgebildet und derartig geformt sind, dass im Hochtemperaturreaktor im Bereich des Gasauslasses (15) niedergeschlagene Salze und/oder Metalloxide mindestens teilweise aufgrund der Schwerkraftwirkung und/oder mit dem Gasstrom in die Filtereinheit (3), insbesondere in den den ersten Bereich der Filtereinheit, gelangen.

6. Vorrichtung nach einem der Ansprüche 2 bis 5,
wobei die Filtereinheit (3) im ersten Bereich eine gegen die Umgebung hermetisch abgedichtete Filterkammer (17) zur Aufnahme von resublimiertem und/oder sedimentiertem Salz und/oder sedimentiertem Metalloxid umfasst.

7. Vorrichtung nach Anspruch 6,
wobei die Filterkammer (17) im Wesentlichen aus einem mit dem Gasauslass (15) oder der Gasleitung vom Gasauslass (15) zur Filtereinheit (3), insbesondere über eine Schnellkupplung, lösbar verbundenen Kammerdeckel (16) und eine lösbar mit dem Kammerdeckel (16) verbundene Wanne (18) gebildet wird.

8. Vorrichtung nach einem der Ansprüche 2 bis 7,
wobei die Filtereinheit (3) im zweiten Bereich einen Feinfilter (19) aufweist, welcher die Filtereinheit (3), insbesondere das Innere der Filterkammer (17), gegenüber der Kondensationseinheit (25) gasdurchlässig abschließt.

9. Vorrichtung nach Anspruch 8,
wobei der Feinfilter (19) ein Filtermittel mit einer Struktur umfasst, welche derart ausgestaltet ist, dass die Abscheidung der Salze und/oder Metalloxide nach dem Prinzip der Tiefenfiltration im Inneren des Filtermittels erfolgt.

10. Vorrichtung nach Anspruch 8 oder 9,
wobei der Feinfilter (19) ein PTFE-Sinterfilterelement, ein Aktivkohlefilterelement, PP-Spinnfasern oder ein Edelstahlgewebe umfasst, und wobei die mittlere Porengröße des Feinfilters 0,4 bis 100 µm, insbesondere 5 bis 25 µm beträgt.

11. Vorrichtung nach einem der Ansprüche 8 bis 10,
wobei in Richtung des Gasstroms vor dem Feinfilter (1), insbesondere innerhalb des Gaseinlasses (6) zur Zuführung des Trägergases, ein Drucksensor angeordnet ist.

## Claims

1. Apparatus (1) for determining the content of at least one oxidizable ingredient in an aqueous liquid sample, comprising a high-temperature reactor for digesting the liquid sample and forming a gas mixture which contains at least the ingredient as a gaseous oxide,
wherein the high-temperature reactor comprises a liquid inlet (5) for supplying the liquid sample and a gas inlet (6) for supplying a carrier gas, and
is connected via a gas outlet (15) to an analysis chamber (35),
wherein a condensation unit (25) for condensing water from the gas mixture is connected upstream of said analysis chamber (35),
wherein the apparatus is configured such that, during operation, a gas flow of the carrier gas is formed with the gas mixture from the high-temperature reactor via the gas outlet (15) and the condensation unit (25) into the analysis chamber (35),
wherein a heatable filter unit (3) for removing salts and/or metal oxides from the gas mixture is interconnected between said gas outlet (15) and said condensation unit (25),
**characterized in that** the gas outlet (15), the filter unit (3) and connecting elements arranged between the gas outlet (15) and the filter unit (3) are thermally insulated and/or equipped with heating elements in such a way that the temperature thereof can be set to more than 100°C during operation of the apparatus (1).

2. Apparatus according to claim 1,
wherein the filter unit (3) comprises a first region which is configured to remove salts and/or metal oxides from the gas mixture by resublimation and/or sedimentation and a second region, downstream of said first region, which is configured to remove salts and/or metal oxides from the gas mixture by retention in a filter medium.

3. Apparatus according to claim 1 or 2,
wherein the filter unit (3) can be heated by means of at least one heating element to an operating temperature above the condensation temperature of water, in particular to a temperature between 100°C and 150°C.

4. Apparatus according to one of claims 1 to 3,
wherein the gas outlet (15) and the filter unit (3) are connected to each other via a thermally insulated and/or heated gas line as a connecting element.

5. Apparatus according to one of claims 1 to 4,
wherein the gas outlet (15) and/or the gas line via which the gas outlet (15) is connected to the filter unit (3) is configured with a wide lumen, in particular with a diameter of more than 3 mm, and is formed in such a way that salts and/or metal oxides deposited in the high-temperature reactor in the region of said gas outlet (15) reach the filter unit (3), in particular the first region of said filter unit, due at least in part to the effect of gravity and/or with the gas flow.

6. Apparatus according to one of claims 2 to 5,
wherein in the first region the filter unit (3) comprises a filter chamber (17) hermetically sealed against the environment for receiving resublimated and/or sedimented salt and/or sedimented metal oxide.

7. Apparatus according to claim 6,
wherein the filter chamber (17) is formed essentially of a chamber cover (16) releasably connected to the gas outlet (15) or the gas line from the gas outlet (15) to the filter unit (3), in particular via a quick-release coupling, and a trough (18) releasably connected to the chamber cover (16).

8. Apparatus according to one of claims 2 to 7,
wherein in the second region the filter unit (3) has a fine filter (19) which closes said filter unit (3), in particular the inside of the filter chamber (17), gas-permeably in relation to the condensation unit (25).

9. Apparatus according to claim 8,
wherein the fine filter (19) comprises a filter medium having a structure which is configured in such a way that separation of the salts and/or metal oxides takes place in the interior of the filter medium according to the principle of deep bed filtration.

10. Apparatus according to claim 8 or 9,
wherein the fine filter (19) comprises a PTFE sintered filter element, an activated charcoal filter element, PP spun fibers or a stainless steel mesh, and wherein the average pore size of the fine filter is 0.4 to 100 µm, in particular 5 to 25 µm.

11. Apparatus according to one of claims 8 to 10,
wherein a pressure sensor is arranged in the direction of the gas flow upstream of the fine filter (19), in particular within the gas inlet (6) for supplying the carrier gas.

## Revendications

1. Dispositif (1) destiné à la détermination de la teneur d'au moins un composant oxydable dans un échantillon liquide aqueux, comprenant un réacteur à haute température destiné à la digestion de l'échantillon de liquide et à la formation d'un mélange gazeux, qui contient le composant sous forme d'oxyde gazeux, le réacteur à haute température présentant une entrée de liquide (5) destinée à l'acheminement de l'échantillon de liquide et une entrée de gaz (6) destinée à l'acheminement d'un gaz porteur, et
qui est relié avec la chambre d'analyse (35) par l'intermédiaire d'une sortie de gaz (15),
une unité de condensation (25), destinée à la condensation d'eau à partir du mélange gazeux, étant disposée en amont de la chambre d'analyse (35),
le dispositif étant conçu de telle sorte que se forme, pendant le fonctionnement, un flux gazeux du gaz porteur avec le mélange gazeux du réacteur à haute température par l'intermédiaire de la sortie de gaz (15) et l'unité de condensation (25) dans la chambre d'analyse (35),
une unité de filtrage (3) chauffante, destinée à l'élimination des sels et/ou des oxydes métalliques se trouvant dans le mélange gazeux, étant insérée entre la sortie de gaz (15) et l'unité de condensation (25),
**caractérisé**
**en ce que** la sortie de gaz (15), l'unité de filtrage (3) et les éléments de liaison disposés entre la sortie de gaz (15) et l'unité de filtrage (3) sont isolés thermiquement et/ou équipés d'éléments chauffants de telle sorte que leur température est réglée, pendant le fonctionnement du dispositif (1), à plus de 100°C.

2. Dispositif selon la revendication 1,
pour lequel l'unité de filtrage (3) comprend une première zone, qui est constituée de telle sorte à éliminer du mélange gazeux les sels et/ou les oxydes métalliques par resublimation et/ou sédimentation, et une deuxième zone disposée en aval de la première zone, qui est conçue de telle sorte à éliminer du mélange gazeux les sels et/ou les oxydes métalliques par retenue dans un moyen de filtrage.

3. Dispositif selon la revendication 1 ou 2,
pour lequel l'unité de filtrage (3) peut être chauffée au moyen d'au moins un élément chauffant à une température de service supérieure à la température de condensation de l'eau, notamment à une température comprise entre 100 °C et 150 °C.

4. Dispositif selon l'une des revendications 1 à 3,
pour lequel la sortie de gaz (15) et l'unité de filtrage (3) sont reliées entre elles par l'intermédiaire d'une conduite de gaz isolée thermiquement et/ou chauffée en tant qu'élément de liaison.

5. Dispositif selon l'une des revendications 1 à 4,
pour lequel la sortie de gaz (15) et/ou la conduite de gaz, par l'intermédiaire de laquelle la sortie de gaz (15) est reliée avec l'unité de filtrage (3), sont conçues avec un grand diamètre, notamment avec un diamètre supérieur à 3 mm, et formées de telle sorte que les sels et/ou les oxydes métalliques, qui se sont déposés dans la zone de la sortie de gaz (15) du réacteur à haute température, parviennent dans l'unité de filtrage (3), notamment dans la première zone de l'unité de filtrage, au moins partiellement sous l'action de la gravité et/ou avec le flux gazeux.

6. Dispositif selon l'une des revendications 2 à 5,
pour lequel l'unité de filtrage (3) comprend dans la première zone une chambre de filtrage (17) hermétiquement étanche par rapport à l'environnement, destinée à l'absorption du sel resublimé et/ou sédimenté et/ou de l'oxyde métallique sédimenté.

7. Dispositif selon la revendication 6,
pour lequel la chambre de filtrage (17) est constituée pour l'essentiel d'un couvercle de chambre (16) relié de façon amovible, notamment par l'intermédiaire d'un raccord rapide, avec la sortie de gaz (15) ou la conduite de gaz de la sortie de gaz (15) vers l'unité de filtrage (3), et d'un récipient (18) relié de façon amovible avec le couvercle de chambre (16).

8. Dispositif selon l'une des revendications 2 à 7,
pour lequel l'unité de filtrage (3) comporte dans la deuxième zone un filtre fin (19), laquelle zone sépare l'unité de filtrage (3) de façon perméable au gaz, notamment à l'intérieur de la chambre de filtrage (17), par rapport à l'unité de condensation (25).

9. Dispositif selon la revendication 8,
pour lequel le filtre fin (19) comprend un moyen de filtrage avec une structure conçue de telle manière à ce que la séparation des sels et/ou des oxydes métalliques intervient d'après le principe du filtrage en profondeur à l'intérieur du moyen de filtrage.

10. Dispositif selon la revendication 8 ou 9,
pour lequel le filtre fin (19) comprend un élément filtrant fritté en PTFE, un élément filtrant à charbon actif, des fibres discontinues en PP ou une structure en acier inoxydable, et pour lequel la taille moyenne des pores du filtre fin est comprise entre 0,4 et 100 µm, notamment entre 5 et 25 µm.

11. Dispositif selon l'une des revendications 8 à 10,
pour lequel est disposé, dans la direction du flux gazeux en amont du filtre fin (19), notamment à l'intérieur de l'entrée de gaz (6), un capteur de pression destiné à l'acheminement du gaz porteur.
